(19) Europäisches Patentamt
European Patent Office
Office européen des brevets

(11) Veröffentlichungsnummer: **0 010 589**
**B1**

(12) # EUROPÄISCHE PATENTSCHRIFT

(45) Veröffentlichungstag der Patentschrift: **12.01.83**

(51) Int. Cl.³: **C 07 D 251/34**, C 08 G 18/79, C 08 G 18/80, C 09 D 3/72

(21) Anmeldenummer: **79103195.8**

(22) Anmeldetag: **29.08.79**

(54) **Verfahren zur Herstellung von Isocyanuratgruppen aufweisenden Polyisocyanaten und ihre Verwendung als Isocyanatkomponente in Polyurethanlacken.**

(30) Priorität: **08.09.78 DE 2839133**

(43) Veröffentlichungstag der Anmeldung: **14.05.80 Patentblatt 80/10**

(45) Bekanntmachung des Hinweises auf die Patenterteilung: **12.01.83 Patentblatt 83/2**

(84) Benannte Vertragsstaaten: **BE DE FR GB IT NL**

(56) Entgegenhaltungen:
**DE-A-1 667 309**
**DE-A-2 641 380**
**DE-A-2 707 656**
**DE-B-1 150 080**
**DE-B-2 226 191**
**US-A-3 010 963**
**US-A-3 892 687**
**US-A-3 988 267**

(73) Patentinhaber: **BAYER AG, Zentralbereich Patente, Marken und Lizenzen, D-5090 Leverkusen 1, Bayerwerk (DE)**

(72) Erfinder: **Bock, Manfred, Dr., Haydnstrasse 18, D-5090 Leverkusen 1 (DE)**
Erfinder: **Pedain, Josef, Dr., Haferkamp 6, D-5000 Köln 80 (DE)**
Erfinder: **Uerdingen, Walter, Dr., Humperdinck Strasse 41, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur Herstellung von Isocyanuratgruppen aufweisenden Polyisocyanaten und ihre Verwendung als Isocyanatkomponente in Polyurethanlacken

Die vorliegende Erfindung betrifft ein neues Verfahren zur Herstellung von lösungsmittelfreien, Isocyanuratgruppen aufweisenden Polyisocyanaten durch teilweise Trimerisierung der Isocyanatgruppen von Hexamethylendiisocyanat.

Für lösungsmittelfreie bzw. lösungsmittelarme lichtbeständige Polyurethanlacke besteht ein Bedarf an niedrigviskosen, farblosen oder nur wenig verfärbten Polyisocyanaten mit hohem NCO-Gehalt.

Potentiell gut geeignet für diesen Zweck könnten niedrigviskose Polyisocyanate sein, die durch Polymerisierung von Hexamethylendiisocyanat entstehen. Hexamethylendiisocyanat ist ein technisch gut zugängliches Produkt, seine Polymerisation zu einem Polyisocyanat mit Isocyanuratgruppen ist schon lange aus der Literatur bekannt. Für eine Anwendung auf dem Gebiet der lichtbeständigen lösungsmittelfreien bzw. lösungsmittelarmen Lacke sind die nach den bekannten Herstellungsverfahren erhaltenen NCO-Gruppen tragenden Polymerisate des Hexamethylendiisocyanats (im folgenden HDI genannt) aber nicht oder nur bedingt geeignet.

So erfüllen beispielsweise die gemäss DE-OS 2 616 415 erhältlichen HDI-Polymerisate trotz ihrer verhältnismässig niedrigen Viskosität wegen ihrer zu Verfärbungen der Lackschichten Anlass gebenden Eigenfarbe nicht alle an Lackpolyisocyanate für hochwertige Polyurethanlacke zu stellenden Bedingungen. Das gleiche gilt für das in DE-AS 2 226 191 in Kolonne 5, Zeile 55 – Kolonne 6, Zeile 6 beschriebene HDI-Polymerisat. Das letztgenannte HDI-Polymerisat weist im übrigen einen hohen Anteil an dimerisiertem HDI auf. Durch Aufspaltung dieses dimeren HDI entsteht beim Lagern des Polyisocyanats sehr leicht wieder freies HDI, so dass das Produkt nach einiger Zeit einen hohen Anteil an freiem HDI aufweist.

Weitere bekannte Verfahren zur Polymerisation von HDI benutzen Metallverbindungen als Katalysatoren, z.B. Alkali- oder Bleisalze. Diese Katalysatoren haben nach GB-PS 809 809 den Vorteil, dass sie zu Produkten führen, die wenig oder kein Dimeres enthalten. Die Katalyse mit Metallverbindungen hat allerdings mehrere Nachteile. Da die Reaktion recht heftig verläuft, entstehen hochviskose, zum Teil uneinheitliche Produkte mit niedrigem NCO-Gehalt (vgl. US-PS 3 330 828 – Beispiel 6, GB-PS 952 931 – Beispiel 2 oder DE-AS 1 013 869 – Beispiel 4).

Durch die uneinheitlich verlaufende exotherme Reaktion entstehen bei der Metallkatalyse auch Gelteilchen (GB-PS 966 338), die schwierig und erst nach Verdünnen mit Lösemittel zu entfernen sind. Ein wesentlicher Nachteil der in den Veröffentlichungen als Katalysator empfohlenen Metallverbindungen ist ausserdem, dass beim Abstoppen des Katalysators anorganische Salze entstehen, die im Polyisocyanat unlöslich sind und Trübungen verursachen und ebenfalls, wenn überhaupt erst nach Verdünnen mit viel Lösemittel zu entfernen sind.

Auch nach dem Verfahren der US-PS 3 211 703 ist das Reaktionsprodukt bei der Polymerisierung von HDI entweder ein Gel oder liegt in verdünnter Lösung vor, ist also für die Verwendung in lösungsmittelfreien oder lösungsmittelarmen Lakken unbrauchbar. Der US-PS 3 211 703 kann nicht entnommen werden, wie HDI zu einem flüssigen, lösungsmittelfreien Polyisocyanat trimerisiert werden könnte. Das gilt auch für die DE-OS 2 644 684. Dort wird das gleiche Katalysatorsystem aus einem Alkylenoxid und N,N'-Endoäthylenpiperazin, wie in US-PS 3 211 703 insbesondere für die Polymerisation von cycloaliphatischen Isocyanaten empfohlen. Die Lehre der DE-OS 2 644 684 geht im übrigen nicht über die Offenbarung der US-PS 3 211 703 hinaus.

In der DE-OS 2 325 826 wird zur Katalyse der Trimerisierung von Isocyanatgruppen Aziridin oder ein Aziridin-Derivat in Kombination mit einem tert.-Amin empfohlen. Ausser dem Nachteil der Mitverwendung von Lösungsmitteln ist beim Verfahren der letztgenannten OS besonders unvorteilhaft, dass giftige, bekanntliche karzinogene Katalysatoren eingesetzt werden müssen. Von Nachteil ist ferner die lange Inkubationszeit der Polymerisationsreaktion.

Gemäss DE-AS 1 150 080 werden als Katalysatoren zur Trimerisierung von Isocyanaten quaternäre Ammoniumhydroxide als Katalysatoren eingesetzt. Wie aus den Ausführungsbeispielen dieser Veröffentlichung ersichtlich, werden die genannten Katalysatoren praktisch nur zur Polymerisation von aromatischen Isocyanaten verwendet. Versuche, nach der Lehre dieser AS aliphatische Isocyanate und insbesondere HDI zu polymerisieren, führen in Abwesenheit von Lösungsmitteln zu uneinheitlichen Reaktionsprodukten, die als Isocyanatkomponente in hochwertigen Polyurethanlacken unbrauchbar sind. Die schlechte Eignung der in der genannten AS empfohlenen Katalysatoren zur Trimerisierung aliphatischer Isocyanate geht im übrigen aus Beispiel 13 hervor, wo die Trimerisierung von n-Hexadecylisocyanat selbst nach 4tägiger Reaktion lediglich zu einer unter 50% liegenden Ausbeute des entsprechenden Trimerisats führt.

Aus dem bisher gesagten ist ersichtlich, dass bislang noch kein technisch einfach durchführbares Verfahren bekannt geworden ist, welches die problemlose Herstellung von praktisch farblosen, niedrigviskosen, lösungsmittelfreien, Isocyanuratgruppen aufweisenden Polyisocyanaten auf Basis von HDI gestattet. Es war nun die Aufgabe der vorliegenden Erfindung, ein solches Verfahren zur Verfügung zu stellen.

Diese Aufgabe konnte überraschenderweise durch Verwendung von am Stickstoff Hydroxyalkyl-substituierten Ammoniumhydroxiden als Katalysator für die Trimerisierung der Isocyanatgruppen gelöst werden. Diese erfindungsgemäss aufgefundene Lösung der gestellten Aufgabe ist überraschend, da beispielsweise aus der US-PS 3 487 080 entsprechend DE-OS 1 667 309 bekannt war, dass derartige spezielle Ammoniumhydroxide selbst zur Trimerisierung des im Vergleich zu Hexamethylendiisocyanat hochreaktiven Toluylendiisocyanats (TDI) ungeeignet sind. So geht aus Tabelle 3 der genannten US-Patentschrift zweifelsfrei hervor, dass beispielsweise β-Hydroxyäthyl-trimethylammoniumhydroxid als alleiniger Katalysator für die Trimerisierung von TDI ungeeignet, da die Gelierungszeit mehr als 4320 Minuten beträgt. Entsprechend der Lehre der US-PS 3 487 080 war es daher auch unerlässlich, derartige Katalysatoren zusammen mit einer Zweitkomponente einzusetzen. Auch die Lehre der US-PS 3 892 687 bestätigt den durch die US-PS 3 487 080 bzw. die DE-OS 1 667 309 vermittelten Eindruck, dass derartige Hydroxylalkyl-substituierte quaternäre Ammoniumhydroxide zur Trimerisierung von Hexamethylendiisocyanat weitgehend ungeeignet sein müssten. In der US-PS 3 892 687 werden nämlich derartige quaternäre Ammoniumhydroxide als wirksame Katalysatoren für eine Reihe unterschiedlicher Reaktionen empfohlen (Kolonne 2, Zeilen 45–58). Lediglich in den Beispielen 3–7 wird jedoch auf die Verwendung derartiger Verbindungen als Katalysatoren für die Trimerisierung von Isocyanaten eingegangen. Hierbei wird gezeigt, dass die quaternären Ammoniumhydroxide zwar die Trimerisierung von Phenylisocyanat bewirken, jedoch kann ohne weiteres aus diesen Beispielen hergeleitet werden, dass die katalytische Wirkung äusserst gering ist, da selbst nach 2tägiger Einwirkung der Katalysatoren lediglich Ausbeuten von 40–72% erzielt werden. Da es nun aber bekannt ist (DE-AS 1 150 080), dass selbst für aromatische Isocyanate hoch-aktive Trimerisierungskatalysatoren (Beispiel 1) als Trimerisierungskatalysatoren für aliphatische Isocyanate weitgehend ungeeignet sind (Beispiel 13), musste der Leser der US-PS 3 892 687 zwangsläufig zu der Auffassung gelangen, dass die dort empfohlenen quaternären Ammoniumhydroxide als Trimerisierungskatalysatoren für aliphatische Isocyanate völlig ungeeignet sind.

Gegenstand der vorliegenden Erfindung ist somit ein Verfahren zur Herstellung von lösungsmittelfreien, Isocyanuratgruppen aufweisenden Polyisocyanaten einer Viskosität von weniger als 10 000 mPas bei 25 °C und einem Gehalt an monomerem Diisocyanat von weniger als 3 Gew.-% durch teilweise Trimerisierung der Isocyanatgruppen von Hexamethylendiisocyanat in Gegenwart mindestens eines quaternären Ammoniumhydroxids als Katalysator und an sich bekannter Abtrennung von überschüssigem, nicht umgesetztem Diisocyanat und gegebenenfalls mitverwendetem Lösungsmittel, dadurch gekennzeichnet dass man als quaternäre Ammoniumhydroxide, am Stickstoff Hydroxyalkyl-substituierte Ammoniumhydroxide eines Molekulargewichts von bis zu 300 der Formel

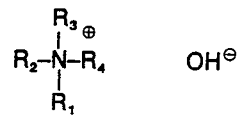

verwendet, wobei

$R_1$ für einen Alkylrest mit 1–20 Kohlenstoffatomen, einen araliphatischen Kohlenwasserstoffrest mit 7–10 Kohlenstoffatomen oder einen gesättigten cycloaliphatischen Kohlenwasserstoffrest mit 4–10 Kohlenstoffatomen steht,

$R_2$, $R_3$ und $R_4$ für gleiche oder verschiedene Reste stehen und Alkylreste mit 1–20 Kohlenstoffatomen bedeuten, wobei 2 der genannten Reste $R_2$, $R_3$ oder $R_3$ auch zusammen mit dem Stickstoff und gegebenenfalls zusammen mit einem Sauerstoff- oder einem weiteren Stickstoff-Heteroatom einen heterocyclischen Ring mit 3–5 Kohlenstoffatomen bilden können, oder wobei die Reste $R_2$, $R_3$ und $R_4$ jeweils für Äthylreste stehen, die zusammen mit dem quatären Stickstoffatom und einem weiteren tertiären Stickstoffatom ein bicyclisches Triäthylen-Diamin-Gerüst bilden können, wobei mindestens einer der genannten Reste $R_1$, $R_2$, $R_3$ oder $R_4$ mindestens eine aliphatisch gebundene Hydroxylgruppe aufweist, und wobei der Hydroxyl-substituierte Rest bzw. die Hydroxyl-substituierten Reste ausser den Hydroxylsubstituenten auch beliebige andere Substituenten aufweisen können.

Beim erfindungsgemässen Verfahren werden somit im Unterschied zum Verfahren der DE-PS 1 150 080 als Katalysatoren am Stickstoff Hydroxyalkyl-substituierte quaternäre Ammoniumhydroxide eingesetzt.

Die bevorzugt beim erfindungsgemässen Verfahren einzusetzenden quaternären Ammoniumhydroxide, sind solche der genannten allgemeinen Formel eines Molekulargewichts von 121–300, für welche

$R_1$ für einen Alkylrest mit 4–12 Kohlenstoffatomen, einen araliphatischen Kohlenwasserstoffrest mit 7 Kohlenstoffatomen oder einen gesättigten cycloaliphatischen Kohlenwasserstoffrest mit 5–6 Kohlenstoffatomen steht,

$R_2$, $R_3$ und $R_4$ für gleiche oder verschiedene Reste stehen und Alkylreste mit 1–4 Kohlenstoffatomen bedeuten, wobei 2 der genannten Reste $R_2$, $R_3$ oder $R_4$ auch zusammen mit dem Stickstoffatom und gegebenenfalls zusammen mit einem Sauerstoff- oder einem weiteren Stickstoff-Heteroatom einen heterocyclischen Ring mit 3–5 Kohlenstoffatomen bilden können, wobei mindestens einer der genannten Reste $R_1$, $R_2$, $R_3$ oder $R_4$ mindestens eine aliphatisch gebundene Hydroxylgruppe aufweist, die in 2-Stellung zum quaternären Stickstoffatom angeordnet ist, wobei der Hydroxyl-substituierte Rest bzw. die Hydroxyl-substituierten Reste ausser den Hydroxyl-Substituenten auch $C_1$–$C_4$-Alkoxy-Substituenten aufweisen können.

Besonders bevorzugte erfindungsgemäss einzusetzende quaternäre Ammoniumhydroxide sind solche der oben genannten Formel, in welchen die Reste $R_1$, $R_2$ und $R_3$ jeweils für Alkylreste der genannten Art und $R_4$ einen Hydroxyäthyl-, Hydro-

xypropyl- oder Hydroxybutylrest bedeuten, in welchem die Hydroxylgruppe vorzugsweise in 2-Stellung zum quaternären Stickstoff angeordnet ist.

Beispiele geeigneter quaternärer Ammoniumhydroxide sind N-(2-Hydroxyäthyl)-N,N-dimethyl-N-(2,2'-dihydroxymethylbutyl)-oammoniumhydroxid, N,N,N-Trimethyl-N-(2-hydroxyäthyl)-ammoniumhydroxid, N-Methyl-2-hydroxyäthyl-morpholiniumhydroxid, N-Methyl-N-(2-hydroxypropyl)-pyrrolidiniumhydroxid, N,N,N-Trimethyl-N-(2-Hydroxypropyl)-ammoniumhydroxid, N,N,N-Trimethyl-N-(2-hydroxybutyl)-ammoniumhydroxid, N-Dodecyl-tris-(2-hydroxyäthyl)-ammoniumhydroxid, Tetra-(2-hydroxyäthyl)-ammoniumhydroxid oder die Verbindung der Formel

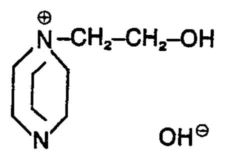

die das Monoaddukt von Äthylenoxid und Wasser an 1,4-Diazabicyclo-[2,2,2]-octan darstellt.

Die erfindungsgemäss einzusetzenden quaternären Ammoniumhydroxide bzw. ihre Herstellung sind bekannt. Ihre Darstellung erfolgt in an sich bekannter Weise durch Umsetzung von Alkylenoxid und tert.-Aminen in wässrigem bzw. wässrigalkoholischem Medium (vgl. US-PS 3 995 997, Kolonne 2, Zeilen 19–44).

Wie bereits ausgeführt erübrigt sich die Mitverwendung eines weiteren Cokatalysators, obwohl prinzipiell die Mitverwendung von an sich bekannten Mannichbasen oder von keine Hydroxyalkyl-Substituenten aufweisenden quaternären Ammoniumhydroxiden als zweite Katalysatorenkomponente im Prinzip möglich jedoch weniger bevorzugt ist.

Die erfindungsgemäss einzusetzenden Katalysatoren werden vorzugsweise in in geeigneten Lösungsmittel gelöster Form eingesetzt. Als Lösungsmittel eignen sich je nach Art des Katalysators beispielsweise Toluol, Dimethylformamid, Dimethylsulfoxid oder auch Mischungen dieser Lösungsmittel. Ebenso ist es möglich, Hydroxyverbindungen als Lösungsmittel für die Katalysatoren zu verwenden, die in Form sogenannter Reaktivverdünner mit in das Isocyanat eingebaut werden. Geeignet sind Monohydroxyverbindungen eines vorzugsweise über 99 liegenden Molekulargewichts wie z.B. Cyclohexanol, 2-Äthylhexanol oder Glykole, wie Äthandiol, Butandiol, Hexandiol, 2-Äthylhexandiol-1,3. Methanol ist jedoch nicht geeignet, da es mit Hexamethylendiisocyanat ein destillativ nicht mehr entfernbares, bei Raumtemperatur auskristallisierendes und daher eine Trübung der Verfahrensprodukte verursachendes Bisurethan bildet. Auf die Abwesenheit von Methanol muss daher bei der Durchführung des erfindungsgemässen Verfahren geachtet werden. Die genannten Lösungsmittel werden im allgemeinen in Mengen von maximal 5 Gew.-%, bezogen auf HDI, eingesetzt. In erster Näherung handelt es sich somit beim erfindungsgemässen Verfahren im allgemeinen um ein «lösungsmittelfreies» Verfahren. Die gegebenenfalls mitverwendeten geringen Mengen an Lösungsmittel werden im Anschluss an die erfindungsgemässe Umsetzung zusammen mit dem überschüssigen Diisocyanat destillativ entfernt, soweit sie nicht in das Verfahrensprodukt eingebaut sind. Die Mitverwendung grösserer Mengen an Lösungsmitteln bei der erfindungsgemässen Umsetzung, die im Anschluss an die Umsetzung destillativ entfernt werden, ist wenig zweckmässig jedoch anderseits auch nicht völlig ausgeschlossen.

Beim erfindungsgemässen Verfahren gelangen die genannten Katalysatoren im allgemeinen in einer Menge von 0,01–0,6 Gew.-%, vorzugsweise von 0,03–0,3 Gew.-%, bezogen auf HDI, zum Einsatz.

Das erfindungsgemässe Verfahren wird im allgemeinen im Temperaturbereich von 10–160 °C, vorzugsweise 20–80 °C, durchgeführt. Die jeweils optimal wirkende Menge an Katalysator richtet sich nach der Art der eingesetzten quartären Ammoniumverbindung.

Die erfindungsgemässe Katalyse bewirkt einen gleichmässigen, im Vorzugsbereich von 20–80 °C in hohem Masse selektiven Trimerisationsverlauf.

Eine Inkubation nach Zugabe von Katalysator tritt nicht auf. Das Temperaturmaximum bei der exothermen Polymerisation (Trimerisierung) wird nicht sprunghaft, sondern allmählich erreicht. Damit ist die Reaktion in jeder Phase gut zu kontrollieren und auch technisch einfach zu beherrschen. Eine optimale Einstellung von Reaktionszeit, Ausbeute sowie Qualität des HDI-Isocyanurats wird durch die Konzentration des Katalysators erreicht. Es hat sich gezeigt, dass durch die erfindungsgemässe sehr schonende Polymerisation über einen Zeitraum von vorzugsweise 2–14 Stunden und anschliessende destillative Entfernung des gegebenenfalls mitverwendeten Lösungsmittels und des überschüssigen Diisocyanats ein praktisch farbloses Reaktionsprodukt mit einer sehr günstigen Viskosität erhalten werden kann. Innerhalb dieses Zeitraums wird ein NCO-Gehalt der Isocyanurat/HDI-Lösung von 34–41%, vorzugsweise 35–40%, angestrebt, so dass Isocyanuratausbeuten zwischen 30–55% erreicht werden. Die Viskosität des Endproduktes liegt dann in jedem Fall unter 10 000 mPas (25 °C). Von Vorteil für das erfindungsgemässe Verfahren ist die Thermoinstabilität des verwendeten Katalysators, so dass die Polymerisierungsreaktion nach Erreichen des gewünschten NCO-Wertes durch Erwärmen der Isocyanuratlösung auf Temperaturen je nach Art der verwendeten Katalysatoren auf 70–120 °C beendet wird. Bei quartären Ammoniumverbindungen mit relativ hoher Zersetzungstemperatur ist es vorteilhaft, der Reaktion Verbindungen zuzusetzen, die die Katalysatoren desaktivieren. Hierzu eignen sich beispielsweise anorganische Säuren wie Salzsäure, Phosphorige Säure oder Phosphorsäure, Carbonsäurehalogenide wie Acetylchlorid oder Benzoylchlorid, Sulfonsäuren oder ihre Derivate wie Methansulfonsäure, p-Toluolsulfonsäure, p-Toluolsulfonsäuremethyl- oder äthylester und perfluorierte Verbindungen, wie beispielsweise die Nonafluorbutansulfonsäure. Im Falle einer chemischen Desaktivierung wird vorzugsweise die letztgenannte Verbindung verwendet. Sie hat gegenüber den üblichen Abstoppern wie Benzoylchlorid oder Estern der p-Toluolsulfonsäure den Vorteil, ohne negativen Einfluss auf die Farbqualität des Endproduktes zu sein. Die Abstopper werden entweder in reiner Form oder gelöst in Lösungsmitteln wie Alkohole oder Dimethylformamid eingesetzt. Überraschenderweise ist im Falle einer Anwendung der genannten Inhibitoren festzustellen, dass zum Abstoppen weit unterhalb der Äquivalenz liegende Mengen von 1–50%, vorzugsweise 5–20%, der Theorie erforderlich sind. Die Vermutung liegt nahe, dass neben der zu erwartenden Desaktivierung durch Salzbildung in übergeordnetem Masse eine durch die Inhibitoren eingeleitete Zerfallsreaktion der verwendeten Katalysatoren in unwirksame Bestandteile stattfindet. Die Beendigung der Trimerisation nach dem erfindungsgemässen Verfahren entweder thermisch oder in der geschilderten Weise durch weniger als äquivalente Mengen an Inhibitoren hat zur Folge, dass Trübungen bei den erfindungsgemässen Verfahrensprodukten selbst in grösseren Verdünnungen mit den in der Lacktechnik gebräuchlichen Lösungsmitteln nicht auftreten. Bevorzugt wird bei der Durchführung des erfindungsgemässen Verfahrens auf die Verwendung von Katalysatorengiften verzichtet und der Katalysator, wie beschrieben, thermisch desaktiviert.

Die Tatsache, dass das erfindungsgemässe Verfahren zur Desaktivierung des Katalysatorsystems keinen bzw. nur sehr wenig Abstopper benötigt, ist gegenüber allen anderen bekannten Verfahren ein wesentlicher Vorteil.

Für die Durchführung des erfindungsgemässen Verfahrens ist von Bedeutung, dass möglichst reines HDI eingesetzt wird, das am besten destilliert und farblos ist.

Im folgenden werden einige allgemeine Arbeitsvorschriften für das erfindungsgemässe Verfahren gegeben:

1. HDI wird in einem Dreihalskolben unter Stickstoffatmosphäre vorgelegt und bei 20–25 °C unter Rühren nacheinander mit den entsprechenden Mengen an Katalysator und versetzt.

Mit Zugabe der Ammoniumverbindung setzt die Trimerisation ein, wobei die Temperatur zunächst innerhalb von 15 Minuten auf 30–35 °C und hiernach langsam innerhalb von 30–90 Minuten auf 40–60 °C ansteigt. Diese Temperatur wird während der Trimerisation aufrechterhalten. Die Abnahme des NCO-Gehaltes wird durch Titration bestimmt. Nach 4–6 Stunden, wenn der angestrebte NCO-Wert erreicht ist, wird die Trimerisation entweder durch kurzzeitiges Erhitzen auf 120 °C oder durch Zugabe einer geringen Inhibitormenge beendet. Die klare, fast farblose Flüssigkeit wird hiernach durch Dünnschichtdestillation im Hochvakuum aufgearbeitet und von überschüssigem HDI und gegebenenfalls mitverwendetem Lösungsmittel befreit. Als Endprodukt erhält man ein farbloses oder schwach gelb gefärbtes, klares Polyisocyanat.

2. Bei einer weiteren Verfahrensvariante gelangen HDI und Katalysator in einem für die Trimerisation geeigneten Verhältnis über Dosierpumpen in eine Mischkammer und von hier in eine Reaktionskaskade mit einer vorgegebenen Temperatur von 50–70 °C, vorzugsweise 55 °C. Der angestrebte NCO-Gehalt, der sich rasch in der Kaskade einstellt, wird vorzugsweise über die Katalysatordosierung geregelt. Im letzten Teil der Kaskade wird im entsprechenden Verhältnis eine Abstopperlösung eingetragen oder die Reaktion auf thermischem Weg durch Erhitzen auf 120 °C beendet. Von hier gelangt die Polymerisatlösung in einen Dünnschichtverdampfer zur Abtrennung des überschüssigen HDI.

Die beim erfindungsgemässen Verfahren erhaltenen Verfahrensprodukte weisen bei 25 °C eine unter 10 000 mPas liegendes Viskosität und einen NCO-Gehalt von 18–24, insbesondere von 20–23 Gew.-% auf.

Das erfindungsgemässe Verfahren hat gegenüber dem Stand der Technik wesentliche Vorteile:

Die dem Verfahren zugrundeliegende Polymerisationsreaktion kann bei einer niedrigen Temperatur schwach exotherm in kurzer Zeit durchgeführt werden, d.h. sie ist energiesparend und gleichzeitig sehr leicht zu beherrschen. Die Katalysatoren können thermisch bzw. mit einer sehr kleinen Menge eines Abstoppers, die weniger als der äquivalenten Menge entspricht, desaktiviert werden. Die Verfahrensprodukte sind stabil und spalten beim Lagern kein freies HDI ab, da sie praktisch keine Uretdion-Gruppen enthalten. Es ist praktisch farblos und weist einen hohen NCO-Gehalt und eine niedrige Viskosität auf. Insbesondere die geringe Eigenfarbe der erfindungsgemässen Verfahrensprodukte ermöglichen ihren Einsatz als Isocyanat-Komponente in hochwertigen Polyurethanlacken. Besonders gut geeignet sind die erfindungsgemässen Verfahrensprodukte für die Herstellung von lösungsmittelfreien bzw. -armen Ein- und Zweikomponenten-Polyurethanlacken. Dabei können die erfindungsgemässen Verfahrensprodukte sowohl als solche als auch in mit Blockierungsmitteln für Isocyanatgruppen blockierter Form zum Einsatz gelangen.

Bevorzugte Reaktionspartner für die erfindungsgemässen, gegebenenfalls in blockierter Form vorliegenden Verfahrensprodukte bei der Herstellung von Polyurethanlacken sind die in der Polyurethanlacktechnik an sich bekannten Polyhydroxypolyester und -äther, Polyhydroxypolyacrylate und gegebenenfalls niedermolekularen, mehrwertigen Alkohole. Auch Polyamine, insbesondere in blockierter Form als Polyketimine oder Oxazolidine sind denkbare Reaktionspartner für die erfindungsgemässen Verfahrensprodukte. Die Mengenverhältnisse, in welchen die erfindungsgemässen, gegebenenfalls blockierten Polyisocyanate und die genannten Reaktionspartner bei der Herstellung von Polyurethanlacken umgesetzt werden, werden im allgemeinen so gewählt, dass auf eine (gegebenenfalls blockierte) Isocyanatgruppe 0,8–3, vorzugsweise 0,9–1,1, Hydroxyl-, Amino-, und/oder Carboxylgruppen entfallen.

Zur Beschleunigung der Aushärtung können in bekannter Weise die in der Isocyanatchemie üblichen Katalysatoren verwendet werden, wie z.B. tert.-Amine wie Triäthylamin, Pyridin, Methylpyridin, Benzyldimethylamin, N,N-Dimethylaminocyclohexan, N-Methylpiperidin, Pentamethyldiäthylentriamin, N,N'-Endoäthylenpiperazin, N,N'-Dimethylpiperazin usw., Metallsalze wie Eisen-(III)-chlorid, Zinkchlorid, Zink-2-äthylcaproat, Zinn(II)-2-äthylcaproat, Dibutylzinn(IV)-dilaurat, Molybdänglykolat usw.

Bei Verwendung der erfindungsgemässen Verfahrensprodukte in Einbrennlacken werden die NCO-Gruppen ganz oder teilweise in bekannter Weise blockiert. Das Polyisocyanat wird mit einem geeigneten Blockierungsmittel, vorzugsweise bei erhöhter Temperatur (z.B. 40–140 °C) gegebenenfalls in Gegenwart eines geeigneten Katalysators, wie z.B. tert.-Amine, Metallsalze wie Zink-2-äthylcaproat, Zinn(II)-2-äthylcaproat, Dibutylzinn(IV)-dilaurat oder Alkaliphenolat umgesetzt.

Geeignete Blockierungsmittel sind beispielsweise:

Monophenole wie Phenol, die Kresole, die Trimethylphenole, die tert.-Butylphenole; tertiäre Alkohole wie tert.-Butanol, tert.-Amylalkohol, Dimethylphenylcarbonol; leicht Enole bildende Verbindungen wie Acetessigester, Acetylaceton, Malonsäurederivate wie Malonsäurediester mit 1–8 C-Atomen in den Alkoholresten; sekundäre aromatische Amine wie N-Methylanilin, die N-Methyltoluidine, N-Phenyltoluidin, N-Phenylxylidin; Imide wie Succinimid; Lactame wie ε-Caprolactam, δ-Valerolactam; Oxime wie Butanonoxim; Cyclohexanonoxim; Mercaptane wie Methylmercaptan, Äthylmercaptan, Butylmercaptan, 2-Mercaptobenzthiazol, α-Naphthylmercaptan, Dodecylmercaptan, Triazole wie 1H-1,2,4-Triazol.

Zur Herstellung der Lackbindemittel werden gegebenenfalls blockiertes Polyisocyanat, polyfunktioneller Reaktionspartner, Katalysator und gegebenenfalls die üblichen Zusätze, wie z.B. Pigmente, Farbstoffe, Füllstoffe und Verlaufmittel miteinander auf einem üblichen Mischaggregat, wie z.B. auf einer Sandmühle entweder mit oder ohne Lösungs- und Verdünnungsmittel gut vermischt und homogenisiert.

Die Anstrich- und Überzugsmittel können in Lösung oder aus der Schmelze, oder in fester Form nach den üblichen Methoden wie z.B. Streichen, Rollen, Giessen, Spritzen, dem Wirbelsinterverfahren oder dem elektrostatischen Pulversprühverfahren auf den zu beschichtenden Gegenstand aufgebracht werden.

Die die erfindungsgemässen Polyisocyanate enthaltenden Lacke ergeben Filme, die überraschend gut auf metallischem Untergrund haften, besonders lichtecht, wärmefarbstabil und sehr abriebfest sind. Darüber hinaus zeichnen sie sich durch grosse Härte, Elastizität, sehr gute Chemikalienbeständigkeit, hohen Glanz, ausgezeichnete Wetterbeständigkeit und gute Pigmentierbarkeit aus.

Die folgenden Beispiele erläutern die Erfindung. Alle Prozentangaben betreffen Gewichtsprozente.

In den folgenden Beispielen werden Katalysatorlösungen I, II, III eingesetzt. Die Herstellung dieser Katalysatoren erfolgt in einem wässrigen Medium wie in US-PS 3 995 997 angegeben. Durch schonende Destillation im Hochvakuum wurden Wasser und andere flüchtige Bestandteile von den Ammoniumhydroxiden abgetrennt und diese danach in 2-Äthylhexanol aufgenommen.

Katalysatorlösung I

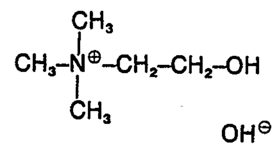

3%ig in Ethylhexanol

Katalysatorlösung II

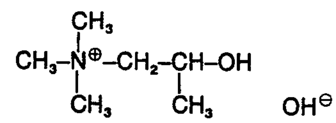

3%ig in Ethylhexanol

Katalysatorlösung III

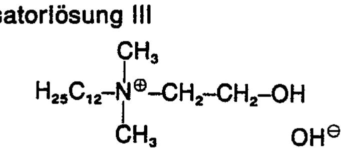

3,5%ig in 2-Ethylhexanol

Beispiel 1

50,5 kg Hexamethylendiisocyanat werden bei 40 °C vorgelegt und mit 750 ml Katalysatorlösung I versetzt. Nach Abklingen der schwach exothermen Reaktion (Temperaturanstieg auf 43–48 °C, 1 Stunde) beträgt der NCO-Gehalt der Flüssigkeit ca. 47%. Das Reaktionsgefäss wird nun auf 47-50 °C geheizt, wobei sich im Reaktionsgefäss eine Temperatur von 53–56 °C einstellt. Der NCO-Gehalt des Produkts nimmt nur in der Stunde um ca. 1,2% ab.

Bei einem NCO-Gehalt von 40–41% wird die Reaktion durch schnelles Aufheizen des Gemischs auf 120 °C unterbrochen.

Nach Filtration des Rohprodukts und Dünnschichtdestillation erhält man 15 kg eines farblosen flüssigen Polyisocyanats. NCO-Gehalt 21,8%, Viskosität (25 °C): 2100 mPas, Gehalt an monomerem HDI: 0,3%.

Beispiel 2

In eine Kaskade, bestehend aus einem Mischgefäss und zwei beheizbaren Reaktionsgefässen werden gleichzeitig 1,344 kg HDI und 45 ml der Katalysatorlösung II eingepumpt. Im Mischgefäss stellt sich eine Temperatur von 35 °C ein, während das erste Reaktionsgefäss auf 55 °C und das zweite auf 60 °C geheizt wird. Während der kontinuierlich ablaufenden Reaktion wird der Verlauf der Polymerisation durch Bestimmungen des NCO-Gehalts und des Brechungsindex kontrolliert. Im ersten Reaktionsgefäss stellt sich ein NCO-Gehalt von 44,5–45,5% ein, im zweiten Reaktionsgefäss ein NCO-Gehalt von 38–40%.

Die mittlere Verweilzeit in der Kaskade beträgt 4 Stunden. Das Rohprodukt wird über einen Vorverdampfer und eine Destillationsapparatur ausgetragen. Im Vorverdampfer wird bei einer Temperatur von 100–120 °C der Katalysator thermisch desaktiviert und ein Teil des überschüssigen HDI abdestilliert. In der Dünnschichtdestillationsapparatur wird bei einer Temperatur von 140–160 °C im Vakuum restliches HDI abdestilliert.

Das Produkt dieses kontinuierlichen Herstellungsprozesses ist praktisch farblos. Es ist klar und dünnflüssig.
NCO-Gehalt: 21,0%
monomeres HDI: 0,25%
Viskosität (25 °C): 3200 mPas.

Beispiel 3

Wie im Beispiel 1 werden 1344 g Hexamethylendiisocyanat bei 45 °C vorgelegt und durch vermischen mit 50 g Katalysatorlösung III die Trimerisierungsreaktion eingeleitet. Innerhalb von 2 Stunden steigt die Temperatur auf das Maximum von 57 °C. Es wird danach noch 2 Stunden auf 60 °C nacherhitzt und noch weitere 5 g Katalysatorlösung III zugefügt. Der NCO-Gehalt des Rohprodukts beträgt dann 41%. Bei weiterem Erhitzen ist keine Abnahme des NCO-Gehalts mehr zu beobachten.

Nun wird die Hälfte der Reaktionsmasse schnell aufgeheizt und zur thermischen Abstoppung von Katalysatorresten 15 Minuten bei 100 °C gehalten. Anschliessend wird überschüssiges HDI über einen Dünnschichtverdampfer abdestilliert. Das Sumpfprodukt ist klar und hat eine schwach gelbliche Färbung.
NCO-Gehalt: 21,9%
Viskosität (25 °C): 3000 mPas
Gehalt an monomerem HDI: 0,5%.

Die zweite Hälfte des Rohprodukts wird mit der Lösung von 0,2 g Nonafluorbutansulfonsäure in 1 ml Dimethylformamid versetzt und dann der Dünnschichtdestillation unterworfen. Auch nach dieser Verfahrensweise erhält man ein dünnflüssiges, hell gefärbtes Produkt mit einem NCO-Gehalt von 22,1%.

**Patentansprüche**

1. Verfahren zur Herstellung von lösungsmittelfreien, Isocyanuratgruppen aufweisenden Polyisocyanaten einer Viskosität von weniger als 10 000 mPas bei 25 °C und einem Gehalt an monomerem Diisocyanat von weniger als 3 Gew.-% durch teilweise Trimerisierung der Isocyanatgruppen von Hexamethylendiisocyanat in Gegenwart mindestens eines quaternären Ammoniumhydroxids als Katalysator und an sich bekannter Abtrennung von überschüssigem, nicht umgesetztem Diisocyanat und gegebenenfalls mitverwendetem Lösungsmittel, dadurch gekennzeichnet, dass man als quaternäre Ammoniumhydroxide, am Stickstoff Hydroxyalkyl-substituierte Ammoniumhydroxide eines Molekulargewichts von bis zu 300 der Formel

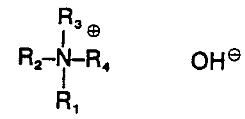

verwendet, wobei

$R_1$ für einen Alkylrest mit 1–20 Kohlenstoffatomen, einen araliphatischen Kohlenwasserstoffrest mit 7–10 Kohlenstoffatomen oder einen gesättigten cycloaliphatischen Kohlenwasserstoffrest mit 4–10 Kohlenstoffatomen steht,

$R_2$, $R_3$ und $R_4$ für gleiche oder verschiedene Reste stehen und Alkylreste mit 1–20 Kohlenstoffatomen bedeuten, wobei 2 der genannten Reste $R_2$, $R_3$ oder $R_3$ auch zusammen mit dem Stickstoff und gegebenenfalls zusammen mit einem Sauerstoff- oder einem weiteren Stickstoff-Heteroatom einen heterocyclischen Ring mit 3–5 Kohlenstoffatomen bilden können, oder wobei die Reste $R_2$, $R_3$ und $R_4$ jeweils für Äthylreste stehen, die zusammen mit dem quatären Stickstoffatom und einem weiteren tertiären Stickstoffatom ein bicyclisches Triäthylen-Diamin-Gerüst bilden können, wobei mindestens einer der genannten Reste $R_1$, $R_2$, $R_3$ oder $R_4$ mindestens eine aliphatisch gebundene Hydroxylgruppe aufweist, und wobei der Hydroxyl-substituierte Rest bzw. die Hydroxyl-substituierten Reste ausser den Hydroxylsubstituenten auch beliebige andere Substituenten aufweisen können.

2. Verfahren gemäss Anspruch 1, dadurch gekennzeichnet, dass man die Trimerisierungsreaktion nach Erreichen des gewünschten NCO-Wertes durch Erwärmen auf 70–120 °C abbricht.

3. Verwendung der gemäss Anspruch 1 und 2 erhaltenen Verfahrensprodukte, gegebenenfalls in mit Blockierungsmitteln für Isocyanatgruppen blockierter Form als Isocyanatkomponente in Polyurethanlacken.

**Claims**

1. Process for the preparation of solvent-free polyisocyanates which contain isocyanurate groups and have a viscosi of less than 10 000 mPas at 25 °C and a content of monomeric diisocyanate of less than 3% by weight by partial trimerisati of the isocyanate groups of hexamethylene diisocyanate in the presence of at least one quaternary ammonium hydroxide as catalyst and by separation known per se of excess, unreacted diisocyanate and solvent which may have been used, characterised in that the quaternary ammonium hydroxides used are ammonium hydroxides which are hydroxylalkyl-substituted at the nitrogen atom and have a molecular weight of up to 300 of the formula

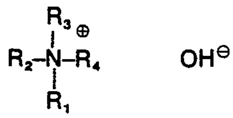

wherein

$R_1$ represents an alkyl radical with 1–20 carbon atoms, an araliphatic hydrocarbon radical with 7–10 carbon atoms or a saturated cycloaliphatic hydrocarbon radical with 4–10 carbon atoms,

$R_2$, $R_3$ and $R_4$ represent identical or different radicals and denote alkyl radicals with 1–20 carbon atoms, wherein 2 of the indicated radicals $R_2$, $R_3$ or $R_3$ together with the nitrogen atom and optionally together with an oxygen hetero-atom or another nitrogen hetero-atom can also form a heterocyclic ring with 3–5 carbon atoms, or wherein the radicals $R_2$, $R_3$ and $R_4$ each represent ethyl radicals which together with the quaternary nitrogen atom and a further tertiary nitrogen atom can form a bicyclic triethylenediamine framework, wherein at least one of the indicated radicals $R_1$, $R_2$, $R_3$ or $R_4$ contains at least one aliphatically bonded hydroxyl group, and wherein the hydroxyl-substituted radical or the hydroxyl-substituted radicals can also contain other substituents in addition to the hydroxyl substituents.

2. Process according to Claim 1, characterised in that the trimerisation reaction is terminated by heating to 70–120 °C after the desired NCO value is reached.

3. Use of the process products obtained according to Claim 1 and 2, optionally in a form blocked by blocking agents for isocyanate groups, as isocyanate component in polyurethane lacquers.

**Revendications**

1. Procédé pour la fabrication de polyisocyanantes à groupes isocyanurates exempts de solvant, d'une viscosité de moins de 10 000 mPas à 25 °C et d'une teneur en diisocyanate monomère de moins de 3% en poids, par trimérisation partielle des groupes isocyanates de l'hexaméthylènediisocyanate en présence d'au moins un hydroxyde d'ammonium quaternaire comme catalyseur et séparation de manière connue du diisocyanate non transformé en excès et éventuellement du solvant utilisé simultanément, caractérisé en ce que l'on utilise comme hydroxydes d'ammonium quaternaire des hydroxydes d'ammonium hydroxyalkyl-substitués à l'azote d'un poids moléculaire allant jusqu'à 300, de formule

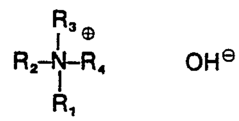

dans laquelle

$R_1$ représente un reste alkyle en $C_1$–$C_{20}$, un reste d'hydrocarbure arylaliphatique en $C_7$–$C_{10}$ ou un reste d'hydrocarbure aliphatique saturé en $C_4$–$C_{10}$,

$R_2$, $R_3$ et $R_4$ sont identiques ou différents et représentent des restes alkyles en $C_1$–$C_{20}$, deux des restes $R_2$, $R_3$ ou $R_3$ mentionnés pouvant aussi former avec l'azote, et éventuellement avec un hétéroatome d'oxygène ou un autre hétéroatome d'azote, un noyau hétérocyclique en $C_3$–$C_5$, ou bien les restes $R_2$, $R_3$ et $R_4$ représentent chaque fois des restes éthyles, qui peuvent former avec l'atome d'azote quaternaire et un autre atome d'azote tertiaire un squelette triéthylènediamine bicyclique, l'un au moins des restes $R_1$, $R_2$, $R_3$ ou $R_4$ mentionnés présentant au moins un groupe hydroxyle à liaison aliphatique et le reste hydroxyl-substitué ou les restes hydroxyl-substitués pouvant comporter aussi, en plus des substituants hydroxyles, d'autres substituants quelconques.

2. Procédé selon la revendication 1, caractérisé en ce qu'on arrête la réaction de trimérisation par chauffage à 70–120 °C après avoir atteint la valeur de NCO désirée.

3. Utilisation des produits du procédé obtenus selon les revendications 1 et 2, éventuellement sous forme bloquée avec des agents bloquants des groupes isocyanates, comme composant isocyanate dans les laques de polyuréthanes.